Europäisches Patentamt

European Patent Office

Office européen des brevets

(11) Publication number: **0 150 949**
A2

(12) # EUROPEAN PATENT APPLICATION

(21) Application number: **85300249.1**

(22) Date of filing: **14.01.85**

(51) Int. Cl.⁴: **A 61 K 7/50,** A 61 K 7/155, C 11 D 1/14, C 11 D 1/83

(30) Priority: **13.01.84 GB 8400908**

(43) Date of publication of application: **07.08.85**
**Bulletin 85/32**

(84) Designated Contracting States: **AT BE CH DE FR IT LI LU NL SE**

(71) Applicant: **LOUIS MARCEL LIMITED, P.O. Box 17 225 Bath Road, Slough, SL1 4AU (GB)**

(72) Inventor: **Nesbit, Michael R., 168 Eastgate, Louth Lincolnshire, LN11 9AE (GB)**

(74) Representative: **Burford, Anthony Frederick et al, W.H. Beck, Greener & Co. 7 Stone Buildings Lincoln's Inn, London WC2A 3SZ (GB)**

(54) **Improvements in detergent compositions.**

(57) A detergent composition is provided for washing the skin after application of an alkaline sulphur containing depilatory composition. Said detergent composition has a pH of less than 6 and comprises an anionic surfactant that is effective at a pH of less than 6 and has a water-soluble calcium salt, a sequestering agent for calcium and a non-ionic thickener.

EP 0 150 949 A2

## IMPROVEMENTS IN DETERGENT COMPOSITIONS

The present invention relates to a detergent composition that is intended to be used after application to the skin of a depilatory cream having as its active ingredient a sulphur containing depilatory material. It also provides a method for removing body hair using the detergent composition for washing the skin.

Presently available depilatory creams commonly employ as active ingredient a sulphur containing depilatory material such as thioglycollic acid or a salt thereof such as calcium thioglycollate at a pH of about 12.5. The cream is applied to the skin and mechanically removed with the unwanted hair after a few minutes. After all visible traces of the cream have been removed, the present recommendation is that the skin should be washed in plain water. Current soaps or detergents have an alkaline reaction and are inappropriate for use until the skin has recovered, otherwise the user may notice a stinging sensation. But neither water nor currently available detergent materials effectively remove calcium salts such as calcium thioglycollate that are present in the pores of the skin or adsorbed on skin cells and that are responsible for the persistant odour associated with the use of depilatory creams because they break the disulphide bonds in skin keratin. Instead they may produce calcium soaps that are insoluble and are trapped in the pores with thioglycollate residue.

It is an object of the invention to provide a detergent composition that has the same pH as skin (about 6) or less (preferably 5.3-5.8) and is effective in removing insoluble sulphur containing depilatory residues from the skin.

Broadly stated the invention provides a detergent composition for washing the skin after application of an alkaline sulphur containing depilatory composition, said composition having a pH of less than 6 and comprising an anionic surfactant that is effective at a pH of less than 6 and does not form a water-insoluble calcium salt, a sequestering agent for calcium and a non-ionic thickener.

The amount of detergent present in the composition may be about 13.5% w/w and the major anionic surfactant present is preferably an amine salt of a sulphated fatty acid e.g. the monoethanolamine salt of lauryl sulphate. An ethoxylated fatty acid monoethanolamide may be present in minor amounts as foam enhancer as may be a surfactant which is a dimethylamine oxide derivative of an ethoxylated fatty alcohol. The composition may contain up to 8% w/w of sequestering agents such as ethylenediaminetetraacetic acid and its salts (e.g. disodium edetate) and glucoheptanoic acid and its salts (e.g. sodium glucoheptanoate.) A water soluble cellulose based thickener or viscosity adjuster may be used in amounts of up to 10% w/w depending upon the properties required, and hydroxyethyl cellulose is a preferred material. The composition may contain an emollient which is a lanolin derivative e.g. an ethoxylated lanolin alcohol in an amount of up to 20% w/w, and may further contain minor amounts of preservative, colouring and perfume together with antioxidants and UV absorbers. The pH may be adjusted by means of citric acid or another weak organic acid.

The invention further provides a method for removing body hair which comprises applying to an area of the skin a chemical depilatory composition, allowing the composition to react with the hair, removing the loosened hair and washing the area of the skin with a composition as aforesaid.

The invention yet further provides a method for removing residues from the chemical depilation of the skin which comprises washing the skin with a composition as aforesaid.

The depilatory composition may be applied by a roller applicator as described in our co-pending application No.        . It may comprise up to 10% of a sulphur containing depilatory material, 0.5-30% of an emulsifying wax to give an oils-in-water cream, 0.1-5% w/w of an inert cellulosic thickener that disperses in the aqueous phase and sufficient alkali to give a pH of 9.0-12.5.

The depilatory material present in the composition may be derived from a thio compound such as thioglycollic acid, thiolactic acid, 3-mercaptopropionic acid and thioglycerol. The acids may be neutralised with alkali metals or alkaline earths such as lithium, sodium, potassium, calcium, barium and strontium. Calcium thioglycollate is the preferred material because it combines high depilatory activity with low skin irritation.

The depilatory composition is desirably formulated to achieve a low fat content and give hydrophilic properties to the composition so that it wets the skin and is easy to remove. Thus the cream or composition base of the oil in water kind may be formed by incorporating into the composition 0.5-30% of a non-ionic emulsifying wax such as that sold by Henkel under the tradename Dehydag AO which contains 80% cetostearyl alcohol and 20% cetomacrogol. To reduce the harshness of the composition when applied to the skin there may be present other inert emollient waxes and oils such as heavy liquid paraffin in amounts of up to 20% w/w. The thixotropy of the composition is maintained at a minimum in order that it should stabilise at the correct viscosity for roller application and flow correctly through the reservoir and applicator head. A non-ionic thickener is preferably therefore incorporated into the aqueous phase of the composition, and water-soluble cellulosic thickeners such as hydroxyethyl cellulose may be used in amounts of 0.1-5% w/w. The latter material is film forming and protects the composition from yellowing due to atmospheric oxidation on composition drying in air. The pH adjuster is preferably at least in part a solid material in order that it may perform the additional function of influencing the flow properties of the composition, and for this purpose the composition may contain up to 10% w/w of an alkaline earth oxide or hydroxide such as ground calcium oxide together with up to 4% w/w of lithium hydroxide and a minor proportion of an alkali metal hydroxide such as sodium or potassium hydroxide to achieve the preferred pH of about 12.5.

The depilatory composition preferably also contains one or more sequestering agents that are effective for calcium ions at high pH values (i.e. pH 9 and above) and the sequestering agent may comprise a major proportion of sodium glucoheptonate and a minor proportion of HEEDTA or a salt thereof such as the trisodium salt. The composition may also contain incidental ingredients such as perfumes, humectants, antioxidants, U-V absorbers and preservatives.

The above composition may be applied, e.g. to the legs, by means of a roller applicator, left in contact with the skin for 1-15 minutes to soften the hair and then washed off by means of the detergent composition aforesaid.

The invention may be provided in the form of a pack containing in a first container a depilatory composition having as principal active ingredient calcium thioglycollate or another salt of thioglycollic acid and in a second container a detergent composition as previously described. A third container may be included in the pack that contains an after-care composition for restoring natural oils to the skin.

The invention will now be further described with reference to the following Examples:

### EXAMPLE I

A gel composition was made by mixing together the following ingredients:

|  | % w/w |
|---|---|
| Monoethanolamine salt of lauryl sulphate (33% aqueous solution) | 30 |
| Ethoxylated fatty acid monoethanolamide | 3 |
| Alkyl dimethylamine oxide (25% aqueous solution) | 2 |
| Sodium glucoheptonate | 0.5 |
| EDTA disodium salt | 0.2 |
| Citric acid | 0.2 |
| Hydroxyethyl cellulose | 1.6 |
| Ethoxylated lanolin alcohols | 1.0 |
| Perfume | 0.2 |
| Rhodamine B [CI (1971) 45170] | 0.0001 |
| Bronopol | 0.02 |
| Water | qsp 100% |

The resulting material was effective to wash off a residue of thioglycollate based depilatory composition from human skin but was mild and acid stable. It had good foaming ability and readily solubilised calcium salts left behind on the skin.

6

## EXAMPLE II

A hair removal composition for roller application to the skin was made by mixing together the following ingredients (% w/w):

| | |
|---|---|
| Calcium thioglycollate trihydrate | 7.5 |
| Non-ionic emulsifying wax (Dehydrag AO) | 6.0 |
| Liquid paraffin (heavy) | 7.0 |
| Hydroxyethyl cellulose | 0.6 |
| Calcium hydroxide (fine ground) | 2.0 |
| Lithium hydroxide | 1.5 |
| Sodium hydroxide (as required, typically) | 0.1 |
| Sodium glucoheptonate | 1.0 |
| N-hydroxyethyl ethylene diamine triacetic acid trisodium salt solution | 0.1 |
| Perfume | 0.3 |
| Water | qsp 100 |

The above composition was charged into the reservoir of an applicator and was spread onto the skin to give uniform layers that engulfed the hairs and had an effective depilatory action, with minimised harshness to the skin and with minimised abrasive properties

The composition was left in contact with the skin for from 5-15 minutes and then rinsed off with water to remove loosened hair. The skin was then washed using the gel composition of Example 1.

CLAIMS:

1. A detergent composition for washing the skin after application of an alkaline sulphur containing depilatory composition, said composition having a pH of less than 6 and comprising an anionic surfactant that is effective at a pH of less than 6 and does not form a water-insoluble calcium salt, a sequestering agent for calcium and a non-ionic thickener.

2. A composition according to claim 1, having a pH of 5.3 to 5.8.

3. A composition according to claim 1 or 2, wherein the surfactant the monoethanolamine salt of lauryl sulphate or a mixture thereof with a minor amount of an ethoxylated fatty acid monoethanolamide and/or with a dimethylamine oxide derivative of an ethoxylated fatty alcohol, or wherein the surfactant comprises an amine salt of a sulphated fatty acid.

4. A composition according to any preceding claim, wherein the sequestering agent is selected from ethylenediaminetetraacetic acid and salts thereof and glucoheptonoic acid and salts thereof.

5. A composition according to any preceding claim, wherein the thickener is hydroxyethyl cellulose or another water soluble cellulose derivative.

6. A composition according to any preceding claim, further comprising ethoxylated lanolin alcohol or another lanolin derivative as emollient.

7. A composition according to any preceding claim, wherein the pH is adjusted to less than 6 by means of citric acid.

8. A pack comprising in a first container a depilatory composition containing as active ingredient a salt of thioglycollic acid and in a second container a detergent composition as claimed in any of claims 1-7.

9. A method for removing body hair which comprises applying to an area of the skin a chemical depilatory composition, allowing the composition to react with the hair, removing the loosened hair and washing the area of the skin with a composition as claimed in any of Claims 1-7.

10. A method for removing residues from the chemical depilation of the skin which comprises washing the skin with a composite as claimed in any of Claims 1-7.